Europäisches Patentamt

European Patent Office

Office européen des brevets

(11) Numéro de publication : **0 465 360 B1**

(12) **FASCICULE DE BREVET EUROPEEN**

(45) Date de publication du fascicule du brevet :
**25.01.95 Bulletin 95/04**

(51) Int. Cl.⁶ : **H05G 1/46, A61B 6/00**

(21) Numéro de dépôt : **91401835.3**

(22) Date de dépôt : **03.07.91**

(54) **Procédé de détermination automatique de la durée d'exposition d'un film radiographique et système de mise en oeuvre.**

(30) Priorité : **06.07.90 FR 9008625**
**06.07.90 FR 9008628**

(43) Date de publication de la demande :
**08.01.92 Bulletin 92/02**

(45) Mention de la délivrance du brevet :
**25.01.95 Bulletin 95/04**

(84) Etats contractants désignés :
**BE DE ES FR IT NL SE**

(56) Documents cités :
**WO-A-87/01555**
**DE-A- 3 641 992**
**GB-A- 2 004 437**
**US-A- 4 763 343**

(73) Titulaire : **GENERAL ELECTRIC CGR S.A.**
**100, rue Camille-Desmoulins**
**F-92130 Issy les Moulineaux (FR)**

(72) Inventeur : **Heidsieck, Robert**
**Cabinet Ballot-Schmit,**
**7 rue Le Sueur**
**F-75116 Paris (FR)**

(74) Mandataire : **Ballot, Paul Denis Jacques et al**
**Cabinet Ballot-Schmit,**
**7, rue Le Sueur**
**F-75116 Paris (FR)**

EP 0 465 360 B1

## Description

L'invention concerne les systèmes de radiologie comprenant un film radiologique et utilisés pour examiner des objets et, plus particulièrement dans de tels systèmes, un procédé qui permet d'estimer, pendant l'examen de l'objet, la lumination reçue par le film radiologique et d'arrêter l'exposition lorsque le film est arrivé à un niveau de noircissement donné.

Un système de radiologie comprend essentiellement un tube à rayons X et un récepteur d'un tel rayonnement entre lesquels est interposé l'objet à examiner tel qu'une partie du corps d'un patient. Le récepteur image qui est, par exemple, un couple film-écran, fournit après une durée de pose appropriée et développement du film, une image de l'objet. Pour que l'image de l'objet puisse être exploitée dans les meilleures conditions, il faut que les différents points qui constituent cette image présentent entre eux un contraste suffisant, c'est-à-dire que le noircissement du film radiographique soit correct, et cela d'une radiographie à la suivante, malgré les différences d'opacité que peut présenter l'objet radiographié.

Le noircissement du film est lié à la quantité d'énergie du rayonnement incident sur le couple film-écran, c'est-à-dire le produit de l'intensité du rayonnement auquel est soumis le film radiographique, ou débit de dose "film", par le temps durant lequel le film est exposé à ce rayonnement. En conséquence, pour obtenir un noircissement constant du film d'une radiographie à la suivante, il est connu (voir US-A-4 763 343 correspondant à EP-A-0 346 530) de mesurer, au cours de l'examen, l'énergie incidente sur le film au moyen d'une cellule de détection, placée en aval du récepteur, qui est sensible au rayonnement X et qui fournit un courant proportionnel au débit de dose "film". Ce courant est intégré, à compter du début de la pose, dans un circuit intégrateur qui fournit une valeur croissante au cours de la pose. Cette valeur croissante est comparée durant le temps de pose à une valeur de consigne fixe, préalablement établie en fonction des caractéristiques du film. La fin du temps de pose est déterminée par l'instant auquel la comparaison indique que la valeur représentative de l'énergie incidente sur le film est égale à la valeur de consigne.

Dans le cas où le film radiographique est directement soumis au rayonnement X et que la variation des temps de pose d'un examen à l'autre est suffisamment faible, un noircissement constant du film est obtenu d'une pose à la suivante, indépendamment de la durée du temps de pose S, à condition que le produit du temps de pose S par le débit de dose F soit constant, c'est-à-dire que la valeur résultant de l'intégration doit rester constante. Ceci n'est vrai que si les caractéristiques du film obéissent à la loi de réciprocité qui indique que la densité optique du film est proportionnelle au produit F x S et si la réponse du film est indépendante de la qualité du faisceau de rayons X incident.

Cette loi de réciprocité n'est plus vérifiée dans le cas où la variation des temps de pose est forte. Par ailleurs, dans le cas où le film radiographique est associé à un écran renforçateur, le noircissement du film dépend de la qualité du spectre. En effet, la réponse de l'écran dépend de la répartition énergétique du spectre du rayonnement reçu, ce qui signifie qu'il est sensible au durcissement de spectre et au changement de tension du tube à rayons X.

Enfin, il existe certaines applications pour lesquelles il est pénalisant que la cellule de détection soit placée avant le film, par exemple en mammographie, car l'énergie de rayonnement est telle que la cellule de détection serait alors visible sur le film. Dans ce cas, elle est placée derrière le récepteur image mais cela crée une difficulté supplémentaire car le signal perçu par la cellule détectrice est celui qui n'a pas contribué au noircissement du film. Il en résulte que la mesure effectuée par la cellule de détection ne représente pas, en général, la lumination incidente sur le film radiographique.

L'écart à la loi de réciprocité, qui varie selon le type de film, représente la variation relative de la lumination nécessaire pour obtenir une densité optique constante lorsque le temps de pose S varie alors que le spectre du rayonnement X est constant. Ceci se traduit par le fait que pour obtenir une même densité optique du film, la lumination devra être par exemple de 1 pour un temps de pose S = 0,1 seconde, de 1,3 pour S = 1 seconde et de 2 pour S = 4 secondes.

Cet écart à la loi de réciprocité est dû au phénomène connu sous le nom d'effet Schwarzschild. Cet effet est décrit notamment dans le livre intitulé : "CHIMIE ET PHYSIQUE PHOTOGRAPHIQUES" de Pierre GLAFKIDES - 4ème édition, pages 234 à 238 et édité par PUBLICATIONS PHOTO-CINEMA Paul MONTEL.

Pour tenir compte de cet écart à la loi de réciprocité, il a été proposé différentes solutions et l'une d'entre elles a été décrite dans le brevet français 2 584 504 (correspondant à EP-A-0 208 607). Dans ce brevet, il est prévu de comparer la valeur intégrée du signal fourni par la cellule de détection à une valeur de consigne qui varie au cours de la pose selon une loi déterminée. Plus précisément, à partir du début de chaque temps de pose, on ajoute à la différence des valeurs du signal intégré et de la consigne une valeur additionnelle qui est croissante en fonction du temps selon une loi préalablement déterminée, par exemple exponentielle.

Cette loi préalablement déterminée, qu'elle soit exponentielle ou autre, ne rend compte de l'écart à la loi de réciprocité que de manière imparfaite, notamment en ne tenant pas compte des variations de l'intensité

2

lumineuse effectivement reçue par le film.

En outre, cette correction ne tient pas compte des effets d'autres phénomènes tels que le durcissement du rayonnement X dû à l'épaisseur d'objet traversé, à la modification du spectre due à la tension du tube à rayons X.

De plus, dans ce procédé, la cellule de détection est placée avant le récepteur image.

Le but de la présente invention est donc de mettre en oeuvre un procédé pour déterminer automatiquement, pendant la durée du temps de pose, l'instant d'arrêt de la pose en tenant compte des différents effets qui interviennent, notamment les variations du courant de tube, le durcissement du spectre dû à l'épaisseur d'objet traversé, à la modification du spectre due à la tension de tube et de la réponse en absorption d'un écran renforçateur.

Selon une première variante, l'invention concerne un procédé de détermination automatique de la durée d'exposition d'un film radiographique selon la revendication 1.

Selon une deuxième variante, l'invention concerne un procédé de détermination automatique de la durée d'exposition d'un film radiographique selon la revendication 2.

Le récepteur image avec cellule détectrice d'exposeur automatique incorporée est semblable à celui décrit dans la demande de brevet français n° 89 05668 (FR-A-2646515). Il comprend une boîte qui réalise une chambre noire photographique, au moins un écran qui convertit le rayonnement X en un rayonnement lumineux et un film sensible audit rayonnement lumineux qui est disposé en amont dudit écran. Il comprend en outre une cellule détectrice du rayonnement lumineux qui est disposée en aval dudit écran et du film pour, d'une part, détecter le rayonnement lumineux émis par ledit écran et, d'autre part, fournir un ou plusieurs signaux électriques représentatifs du rayonnement lumineux et un dispositif de transmission dudit ou desdits signaux électriques vers le circuit intégrateur.

Les revendications 3 à 15 se rapportent aux réalisations préférées de l'invention.

D'autres buts, caractéristiques et avantages de la présente invention apparaîtront à la lecture de la description suivante du procédé selon l'invention et d'un exemple particulier de réalisation du système de radiologie pour le mettre en oeuvre, ladite description étant faite en relation avec les dessins joints dans lesquels :

- la figure 1 est un schéma fonctionnel d'un système de radiologie qui permet de mettre en oeuvre le procédé selon l'invention,
- la figure 2 est un diagramme montrant des courbes obtenues en mettant en oeuvre un procédé d'étalonnage utilisé dans le procédé selon l'invention,
- la figure 3 est un diagramme montrant une courbe de variation des coefficients de non réciprocité CNRT en fonction du temps de pose t,
- la figure 4 est un diagramme montrant des courbes de variation des coefficients de non réciprocité CNRD en fonction de l'inverse du débit d,
- la figure 5 est un diagramme montrant des courbes de variation de la densité optique d'un film radiographique en fonction de la lumination, et
- la figure 6 est un schéma fonctionnel d'un système de radiologie similaire à celui de la figure 1 mais dans lequel la cellule de détection est incorporée dans le récepteur et est sensible au rayonnement lumineux émis par l'écran renforçateur.

Un système de radiologie auquel s'applique le procédé de détermination automatique du temps de pose d'un objet à radiographier 13 selon l'invention comprend une source 11 de rayonnement X tel qu'un tube à rayons X qui fournit un faisceau 14 de rayons X illuminant cet objet 13 et un récepteur image 17, tel qu'un couple film-écran, qui est placé de manière à recevoir les rayons X ayant traversé ledit objet et qui fournit, après une durée de pose S appropriée et développement du film, une image de l'objet 13.

Pour mettre en oeuvre le procédé de l'invention, le système comprend, en outre, une cellule de détection 12 qui est placée derrière le récepteur image 17 dans le cas d'un film radiographique avec écran renforçateur. Cette cellule peut être placée devant le récepteur dans le cas d'un film sans écran renforçateur. La cellule de détection 12 permet de convertir une grandeur physique caractéristique du rayonnement X ayant traversé l'objet et le récepteur image, tel que le KERMA ou la fluence énergétique, en un signal de mesure L, par exemple de type électrique. Le signal L, fourni par la cellule de détection 12, est appliqué à un circuit 16 qui réalise une intégration du signal électrique pendant la durée S de la pose. Le signal M, qui résulte de l'intégration, est une mesure du rayonnement ayant traversé l'objet 13 pendant la durée S de la pose.

La source 11 de rayonnement X est associée à un dispositif d'alimentation 15 qui fournit une haute tension d'alimentation variable $V_m$ du tube à rayons X et qui comprend un appareil de mesure du courant anodique I dudit tube. Afin de modifier la durée du temps de pose S, le dispositif d'alimentation 15 et le tube à rayons X comprennent des moyens pour démarrer l'émission de rayons X à un instant précis et l'arrêter après une durée S variable qui est déterminée, conformément au procédé de l'invention, en fonction du signal M fourni par le circuit 16 et des valeurs de I, S et $V_m$ et, plus précisément, du rapport M/I x S qui est appelé rendement D et qui est calculé par le dispositif 18. Les valeurs du rendement D sont traitées par un calculateur ou microprocesseur 19 conformément au procédé de l'invention de manière à fournir un signal de fin de pose.

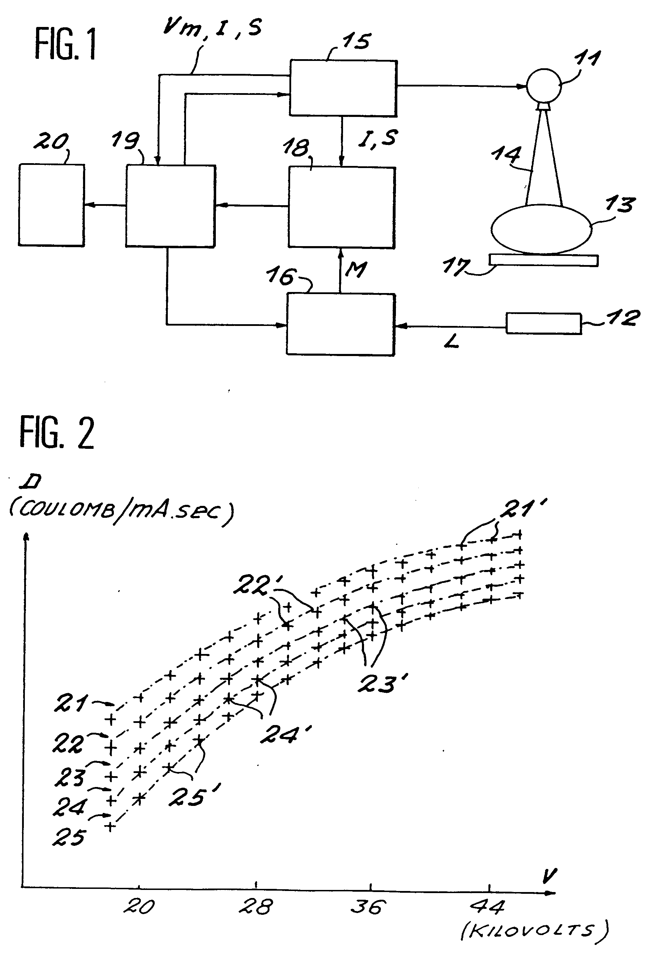

La première opération du procédé consiste à effectuer un étalonnage du système de radiologie de la figure 1 qui aboutit à une fonction d'estimation de la lumination vue par le film radiographique. Cet étalonnage et la fonction d'estimation sont décrits dans la demande de brevet français (correspondant à EP-A-0465362) déposée ce jour et intitulée : "PROCEDE D'ESTIMATION ET D'ETALONNAGE DE LA LUMINATION RECUE PAR UN FILM RADIOGRAPHIQUE".

Pour la compréhension de la suite de la description, on rappellera que le procédé d'estimation de la lumination reçue par un film radiographique est basé sur des étalonnages aboutissant à la définition d'une fonction proportionnelle au débit photonique sur le film, appelée débit-film, et sur un étalonnage permettant de faire le lien entre la fonction débit-film et la lumination reçue par le film dans des conditions de référence fixées et aboutissant à un noircissement donné du film. Ce dernier étalonnage sera plus amplement détaillé dans la suite de la description.

Les étalonnages qui permettent de définir la fonction débit-film sont dérivés d'un procédé d'étalonnage décrit dans la demande de brevet n° 89 07686 (correspondant à EP-A-0402244) déposée le 9 juin 1989 et intitulée " PROCEDE D'ETALONNAGE D'UN SYSTEME RADIOLOGIQUE ET DE MESURE DE L'EPAISSEUR EQUIVALENTE D'UN OBJET ". Ce procédé consiste à mesurer le rendement D de la cellule pour chaque étalon aux tensions d'alimentation $V_m$ choisies. Plus précisément, avec un premier étalon d'épaisseur $E_1$, on effectue une mesure de rendement $D_{1m}$ pour chaque valeur $V_m$ constituant un ensemble déterminé. Ces valeurs $D_{1m}$ en fonction de la tension $V_m$ peuvent être reportées sur un diagramme pour obtenir les points 21' de la figure 2.

Les mesures de rendement D sont effectuées pour un autre étalon d'épaisseur $E_2$ et on obtient les valeurs $D_{2m}$ correspondant aux points 22' de la figure 2 et ainsi de suite pour obtenir les autres séries de points 23', 24' et 25' correspondant respectivement aux rendements $D_{3m}$ $D_{4m}$ et $D_{5m}$ et aux épaisseurs $E_3$, $E_4$ et $E_5$.

Il est à noter que, sur la figure 2, les rendements $D_{pm}$ ont été reportés en ordonnées logarithmiques tandis que les tensions d'alimentation ont été reportées en abscisses de 20 kilovolts à 44 kilovolts.

Ces séries de points 21' à 25' servent à définir les paramètres d'un modèle analytique qui décrit le comportement du rendement D en fonction des paramètres $V_m$ et $E_p$ pour une configuration donnée du système radiologique. Ce modèle analytique sera noté :

$$D \ = \ f\,(V_m,\,E_p) \quad (1)$$

Les paramètres du modèle analytique peuvent être ajustés à l'aide d'outils classiques d'estimation tels que la méthode de minimisation de l'erreur quadratique. Les courbes 21 à 25 représentent la valeur du rendement D donnée par le modèle analytique représenté par l'expression :

$$D \ = \ f\,(V_m,\,E_p) \ = \ \exp\,[f_1\,(V_m) \ + \ E_p \times f_2\,(V_m)] \quad (2)$$

dans laquelle $f_1\,(V_m)$ et $f_2\,(V_m)$ sont des polynômes du deuxième degré dont l'expression est donnée par :

$$f_1\,(V_m) \ = \ A_o \ + \ A_1\,V_m \ + \ A_2\,V_m^2$$

$$f_2\,(V_m) \ = \ B_o \ + \ B_1\,V_m \ + \ B_2\,V_m^2$$

La fonction inverse de celle exprimée par la formule (2) permet de calculer $E_p$ si l'on connaît D et $V_m$ en utilisant la formule (3) suivante :

$$E_p \ = \ g\,(V_m,D) \ = \ \frac{Ln\,(D) \ - \ f_1\,(V_m)}{f_2\,(V_m)} \quad (3)$$

sachant que $f_2\,(V_m)$ ne peut pas s'annuler pour les valeurs courantes de $V_m$ car le rendement D dépend toujours de l'épaisseur $E_p$ aux tensions $V_m$ considérées. En d'autres termes, à un couple de valeurs $(E_p,\,V_m)$ correspond une mesure de rendement D, ce qui permet de déterminer $E_p$ en fonction de $V_m$ et D. Au cours d'un examen radiologique, une mesure de rendement D, qui est effectuée avec une tension d'alimentation $V_m$ donnée, permet de déterminer une épaisseur équivalente exprimée dans les unités utilisées pour $E_p$.

Cet étalonnage est effectué par deux fois avec des configurations différentes du système de radiologie en ce qui concerne le récepteur 17. Le premier de ces étalonnages est réalisé avec le récepteur 17 sans écran renforçateur. Suivant l'équation (1), on détermine une fonction f' qui donne lieu à des valeurs de rendement de la cellule 12 notées $D_{se}$ telles que :

$$D_{se} \ = \ f'\,(V_m,\,E_p) \quad (4)$$

et la fonction inverse :

$$E_p \ = \ g'\,(V_m,\,D_{se}) \quad (5)$$

La deuxième opération du procédé consiste à effectuer un deuxième étalonnage avec un récepteur 17 muni d'un écran renforçateur et on obtient alors une série de valeurs de rendement $D_c$ et on détermine, comme précédemment, la fonction f" telle que :

$$D_c \ = \ f''\,(V_m,\,E_p) \quad (6)$$

et la fonction inverse

$$E_p = g'' (V_m, D_c) \quad (7)$$

Des deux étalonnages précédents, on déduit une fonction $D_f$ qui représente le rendement sur le film tel que :

$$D_f = D_{se} - D_c$$

soit

$$D_f = f'(V_m, E_p) - f''(V_m, E_p) \quad (8)$$

Cette fonction $D_f$ ne tient pas compte de la modification du spectre du rayonnement X due à la filtration additionnelle entre l'écran renforçateur et la cellule de détection 12 qui provient, par exemple, de la face de sortie de la cassette contenant le couple film-écran. Pour en tenir compte, on remplace $E_p$ dans l'équation (8) par ($E_p$ - sup.filtre) où sup.filtre est l'épaisseur équivalente à l'objet radiographié correspondant à cette filtration.

On obtient cette épaisseur équivalente en plaçant, par exemple, dans le faisceau 14 un objet équivalent à cette filtration et en utilisant la fonction étalonnée déterminant l'épaisseur équivalente g' ou g'' suivant la configuration de la machine.

Comme le produit $D_f$ x I x t est proportionnel à l'énergie absorbée dans l'écran renforçateur pendant un temps t et pour un courant anodique I, la quantité $D_f$xI, notée débit-film, est proportionnelle au débit photonique incident sur le film et est exprimée dans les unités de mesure du signal de la cellule détectrice 12. Cette relation de proportionnalité est d'autant mieux vérifiée que le nombre de photons lumineux émis par l'écran renforçateur est lui-même proportionnel à l'énergie absorbée. Si le nombre de photons lumineux émis par l'écran répond à une autre loi en fonction de l'énergie absorbée, il faut appliquer cette autre loi sur $D_f$x I pour obtenir le "débit-film".

Un dernier étalonnage consiste à relier les fonctions électriques précédemment décrites à une valeur du noircissement du film, c'est-à-dire une densité optique, que l'on souhaite obtenir à la fin de la pose. Le choix de cette valeur est effectué par le praticien en fonction du couple film-écran, du type de diagnostic, de la partie du corps du patient à examiner et de ses habitudes d'examen des radiographies. Ce choix permet de déterminer la lumination de référence, notée $L_{ref}$, c'est-à-dire la lumination que doit recevoir le film, dans des conditions de référence fixées, pour arriver à un tel noircissement. Le procédé de détermination de $L_{ref}$ sera décrit ultérieurement. Ces opérations d'étalonnage ne sont pas effectuées à chaque examen radiologique d'un objet ou d'un patient mais seulement une fois de temps à autre pour tenir compte des variations des caractéristiques du système de radiologie au cours du temps, notamment le vieillissement du tube à rayons X. Les résultats de ces opérations sont enregistrés dans la mémoire du microprocesseur 19 sous la forme des fonctions représentées par les équations 4 à 8, ce qui signifie que le microprocesseur 19 sait calculer $E_p$ s'il connaît $D_c$ et peut alors calculer $D_f$.

Au cours de l'examen radiologique du patient, le procédé selon l'invention consiste en outre, à effectuer les opérations principales suivantes :

(e1) mise en place de l'objet ou du patient à radiographier,

(e2) déclenchement du début de la pose par le praticien

(e3) mesure du rendement $D_c$ un certain temps t' après le début de la pose,

(e4) calcul de l'épaisseur équivalente à partir de la mesure de rendement $D_c$,

(e5) calcul du rendement $D_f$ au niveau du film,

(e6) estimation de la lumination reçue par le film depuis le début de la pose,

(e7) calcul de la lumination restant à acquérir pour obtenir le noircissement choisi,

(e8) calcul prévisionnel des mA.s restant à débiter dans le tube à rayons X pour obtenir le noircissement choisi,

(e9) mesure des mA.s, notée $mAs_{mes}$, débités suivant le cas depuis le début de la pose ou de la mesure précédente,

(e10) arrêt du rayonnement X lorsque les $mAs_{mes}$ sont supérieurs ou égaux aux mA.s calculés ou retour à l'opération (e3) dans le cas contraire.

Il est à noter que l'on appelle lumination le produit de la quantité de lumière reçue, par exemple l'éclairement EC de la surface sensible, par la durée de l'exposition ou pose.

L'opération (e3) consiste à mesurer la valeur intégrée D fournie par le dispositif 18 un certain temps t' après le début de la pose sachant que l'intégrateur 16 a été remis à zéro soit, suivant le cas, au début de la pose, soit après la dernière mesure. Le temps t' d'intégration correspond, suivant le cas, au temps écoulé depuis le début de la pose ou au temps écoulé depuis la dernière mesure.

L'opération (e4) est effectuée par le microprocesseur 19 à partir du premier étalonnage du système de radiologie tel que décrit ci-dessus : elle est régie par l'équation (7); on obtient alors une valeur $E_1$ de l'épaisseur équivalente.

Il est à remarquer que pour la deuxième itération du procédé et les suivantes, il n'est pas nécessaire d'effectuer l'opération (e4) dans la mesure où l'estimation de l'épaisseur équivalente a été suffisamment précise lors de la première itération. L'opération (e5) consiste à calculer le rendement du film $D_{f1}$ correspondant à l'épaisseur $E_1$ en utilisant la fonction définie par l'équation (8), ce qui permet de tenir compte, notamment, de l'influence de l'écran du récepteur. Cette opération a été décrite succinctement ci-dessus.

L'opération (e6) consiste à estimer la lumination $L_f$ reçue par le film depuis le début de la pose en appliquant l'équation suivante :

$$L_f = L_{am} + D_{f1} \times \delta mA.s \quad (9)$$

équation dans laquelle $L_{am}$ est la lumination reçue par le film avant l'opération (e3) et $\delta mA.s$ est le nombre de mA.s débités dans le tube pendant le temps $t'$ et est défini par le produit du courant $I$ de tube par le temps d'intégration $t'$.

L'opération (e7) consiste à calculer la lumination restant à acquérir $L_{ra}$ pour obtenir le noircissement choisi; elle est déterminée par l'équation :

$$L_{ra} = L_{ref} - L_f \quad (10)$$

L'opération (e8) consiste à calculer les mA.s restant à débiter pour obtenir le noircissement choisi et qui est donné par l'équation :

$$mAs_r = L_{ra}/D_{f1} \quad (11)$$

Il est alors possible de déduire le nombre de mA.s débités pendant les calculs, noté $mAs_c$. Alors, les mA.s restant réellement à acquérir, notés $mAs_{ra}$, sont définis par :

$$mAs_{ra} = mAs_r - mAs_c \quad (12)$$

où

$$mAs_c = I \times t_c \quad (13)$$

avec $t_c$ le temps des calculs.

L'opération (e10) consiste à effectuer un choix : soit arrêter la pose, soit la continuer selon la valeur des mAs restant à débiter ou encore du temps de pose restant à courir, soit recalculer l'estimation de la valeur prévisionnelle du temps de fin de pose.

Le critère de fin de pose pourra être le suivant :

Si la valeur

$$Dif (mA.s) = mAs_{ra} - mAs_{mes} \quad (15)$$

est nulle ou inférieure à une valeur $Val_0$ fixée, le microprocesseur 19 arrête le rayonnement X par action sur le dispositif d'alimentation 15. Dans le cas contraire, on revient à l'opération (e3).

Il est possible d'envisager un test supplémentaire sur la valeur du temps de pose restant à courir $t_{rc}$ définie par la relation :

$$t_{rc} = \frac{mAs_{ra}}{I} \quad (14)$$

Ce test supplémentaire consiste à ne pas modifier la valeur de l'estimation $mAs_{ra}$ dans le cas où $t_{rc}$ est inférieur à une valeur $t_o$. Alors la fin de la pose se termine en boucle ouverte en ne poursuivant que les opérations de fin de pose, c'est-à-dire la décrémentation du nombre de mA.s débités et arrêt de la pose lorsque ce nombre devient inférieur ou égal à zéro. Une valeur possible de $t_o$ est une valeur sensiblement égale à l'intervalle de temps séparant deux mesures correspondant à l'opération (e3). Ainsi, dans ce cas, l'opération (e10) comporte deux tests :

- un premier test sur $mAs_{ra}$ décidant si on arrête ou non la pose,
- puis un test sur $t_{rc}$ pour décider si on entreprend une nouvelle estimation des mAs restant à débiter ou si la valeur $mAs_{ra}$ reste figée jusqu'à la fin de la pose. Dans ce dernier cas, le test de fin de pose se fera périodiquement avec la valeur $mAs_{ra}$.

Par ailleurs, les opérations d'estimation du temps restant à courir et celle de coupure de la pose peuvent être découplées afin d'affiner encore la précision de l'exposeur. Ainsi, le procédé se décompose de la manière suivante : une tâche T.E. destinée à estimer les mA.s restant à débiter avant la fin de la pose et une tâche T.C. de coupure de la pose. Ce sont deux tâches indépendantes qui se déroulent en parallèle.

La tâche d'estimation T.E. des mA.s restant à débiter est constituée des opérations (e3) à (e8) auxquelles s'ajoute une opération (e'9) de conversion des mA.s en un signal dans les unités de la cellule 12 tel que :

$$CE_{cible} = mAs_{ra} \times D_c \quad (16)$$

Cette tâche d'estimation T.E. est renouvelée périodiquement pendant la pose, par exemple aux instants $t_1, t_2, ... t_n$ qui sont des instants de mesure séparés par une durée qui est au moins égale au temps de calcul $t_c$. A la fin de la tâche d'estimation T.E., la valeur cible $CE_{cible}$ de la tâche de coupure T.C. est actualisée.

Cette actualisation doit tenir compte du signal reçu par la cellule détectrice 12 entre l'instant de mesure au début de l'opération (e3) et l'instant de l'actualisation de la valeur $CE_{cible}$ à la fin de l'opération T.C.

La tâche de coupure de la pose T.C. est une tâche consistant à décrémenter une valeur donnée (ou cible) en fonction du signal réellement reçu par la cellule 12. Cette tâche coupe la pose dès que la valeur $CE_{cible}$ devient inférieure ou égale à $Val_o$, égale à zéro par exemple.

Ainsi, le fonctionnement de la tâche T.C. se résume aux opérations suivantes :

(f1) mesure du signal intégré $M_m$ par la cellule 12 après un certain temps $t_{TC}$;

(f2) décrémentation de cette valeur à la valeur cible : $(CE_{cible} - M_m)$

(f3) arrêt de la pose lorsque $(CE_{cible} - M_m)$ est inférieure à $Val_o$ sinon retour en (f1).

Le procédé qui vient d'être décrit fonctionne correctement dans la mesure où il n'y a pas d'écart à la loi de réciprocité pour le récepteur 17 et la cellule de détection 12. S'il n'en est pas ainsi, il faut compléter les opérations (e6) et (e8) pour en tenir compte et déterminer un coefficient de correction par des mesures et calculs particuliers. Ce coefficient de correction est introduit dans les équations (9) et (11) où interviennent la lumination et le rendement du film.

C'est ainsi que les formules (9) et (11) deviennent :

$$L''_f = L_{am} + D_{f1} \times \delta mA.s/CNRD \text{ (débit - film)} \quad (9')$$

$$mAs''_{ra} = \frac{L_{ra}}{D_{f1}} CNRD \text{ (débit - film)} \quad (11')$$

$$\text{avec débit - film} = D_{f1} \times I \quad (17)$$

CNRD est la fonction représentant l'effet de non réciprocité exprimé en fonction du débit photonique sur le film.

La fonction CNRD est obtenue par un procédé d'étalonnage qui est décrit dans la demande de brevet (EP-A-0465361) déposée ce jour et intitulée : " PROCEDE DE DETERMINATION DE LA FONCTION REPRESENTANT L'EFFET DE NON RECIPROCITE D'UN FILM RADIOGRAPHIQUE".

Pour la compréhension de la suite de la description, on rappellera que ce procédé d'étalonnage consiste d'abord à déterminer les coefficients de non réciprocité du film en fonction de la durée de pose $t_i$ et notés CNRT $(t_i)$. Cette fonction CNRT est déterminée expérimentalement et peut être représentée par une fonction analytique.

De manière plus précise, le procédé consiste à déterminer pour diverses valeurs $IR_i$ de l'intensité du rayonnement, la valeur $t_i$ du temps d'exposition nécessaire pour obtenir une densité optique $D0_{refo}$ du film fixée, par exemple $D0_{refo} = 1$, et à relever les valeurs fournies par le circuit intégrateur 16 pour les différents temps de pose $t_i$, valeurs que l'on appellera $M (t_i)$.

Ces valeurs sont comparées à une valeur de référence $M (t_{ref})$, qui est par exemple celle correspondant à un temps de pose d'une seconde, en calculant le rapport

$$\frac{M (t_i)}{M (t_{ref})} \quad (29)$$

C'est ce rapport qui détermine le coefficient de non réciprocité en temps CNRT $(t_i)$ pour le temps de pose $t_i$.

Une autre manière pour déterminer les coefficients CNRT $(t_i)$ sera décrite ultérieurement.

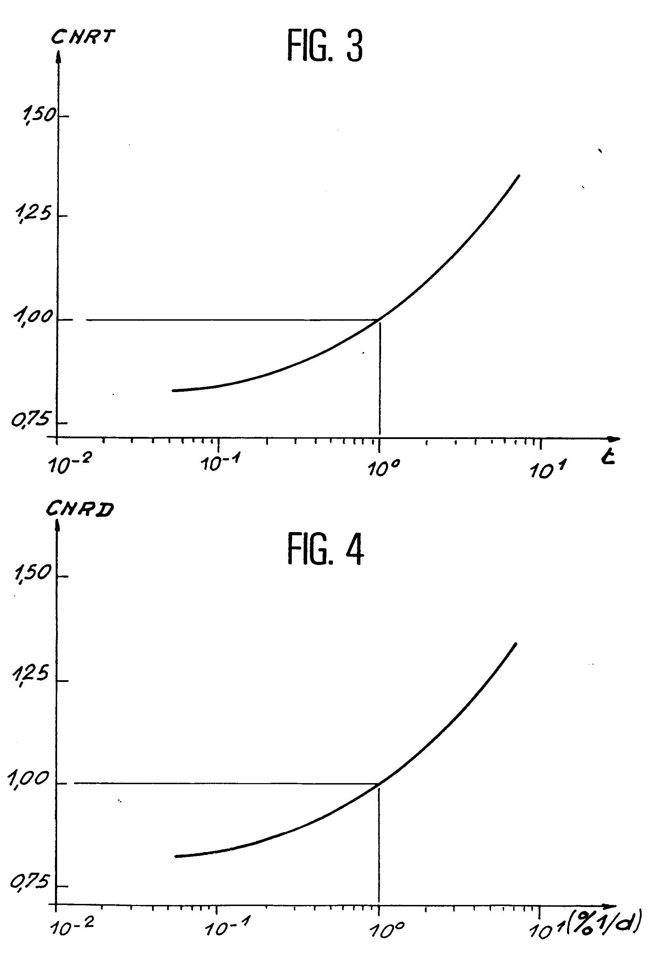

Ces coefficients CNRT $(t_i)$ sont reliés entre eux en fonction du temps de pose par la courbe de la figure 3 dans le cas, par exemple, d'une densité optique $D0_{refo} = 1$ et un temps de pose de référence $t_{ref} = 1$ seconde. Cette courbe montre que la lumination nécessaire pour atteindre la densité optique désirée croît avec le temps de pose. C'est ainsi que, dans cet exemple, le rapport entre les énergies pour les deux temps de pose de 50 ms et 6,5 s est de l'ordre de 1,6.

La courbe de la figure 3 peut être modélisée à l'aide d'une fonction de la forme :

$$CNRT (t_i) = A_o + A_1 \log t + A_2 [\log t]^2 \quad (18)$$

dont les paramètres $A_o$, $A_1$ et $A_2$ sont estimés à partir des points de mesure par une procédure d'estimation aux moindres carrés.

Dans le principe, l'effet Schwarzschild que l'on prend en compte dans les équations (9') et (11') pourrait être modélisé par la fonction CNRT. L'intérêt d'utiliser la fonction CNRD indexée en débit est que l'on peut prendre en compte des variations du courant anodique. Donc, un exposeur automatique qui utilise la fonction CNRD suivant les équations (9') et (11') a, par exemple, pour avantage que le tube peut fonctionner en charge décroissante.

Pour passer des coefficients CNRT (t) indexés en temps aux coefficients CNRD (d) indexés en débit, il faut tenir compte du fait que les coefficients CNRT (t) ont été déterminés par des mesures à temps de pose variable sans connaître nécessairement les valeurs du débit photonique sur le film. Si l'on mesure pour chaque temps de pose $t_i$ le débit-film $d_i$, la valeur du coefficient CNRD $(d_i)$ pour $d_i$ sera égale à celle du coefficient CNRT $(t_i)$ pour le temps de pose $t_i$ correspondant selon la relation :

$$CNRD (d_i) = CNRT (t_i) \quad (19)$$

Ces différentes valeurs de CNRD $(d_i)$ sont reliées entre elles par une courbe (figure 4) en fonction de l'in-

7

verse 1/d du débit. Cette courbe peut être modélisée à l'aide d'une fonction de la forme :

$$CNRD \ (d) \ = \ A'_0 \ + \ A'_1 \ \log \ 1/d \ + \ A'_2 \ \left[ \log \ 1/d \right]^2 \qquad (20)$$

Les valeurs $d_i$ peuvent ne pas être données par l'étalonnage, surtout parce qu'elles sont exprimées dans l'unité de mesure de la cellule 12 qui n'est pas nécessairement celle utilisée dans l'étalonnage. Ainsi, en général, il faut relier les valeurs $d_i$ aux valeurs connues $t_i$ par la relation :

$$L_{ref} \times CNRT \ (t_i) \ = \ d_i \times t_i \quad (21)$$

ou encore :

$$d_i \ = \ \frac{L_{ref} \times CNRT \ (t_i)}{t_i} \quad (22)$$

On rappelle ici que $L_{ref}$ est la lumination que reçoit le film dans des conditions radiologiques fixées et connues lorsque le film atteint un noircissement donné et que l'effet de non réciprocité est corrigé.

Pour finaliser la définition de la fonction CNRD ainsi que pour expliciter le dernier étalonnage du procédé, il reste à exposer la méthode d'évaluation de la lumination de référence.

Cette méthode est décrite dans la demande de brevet précitée (EP-A-0 465 362) et intitulée : PROCEDE D'ESTIMATION ET D'ETALONNAGE DE LA LUMINATION RECUE PAR UN FILM RADIOGRAPHIQUE.

La lumination de référence dépend de la densité optique que l'on souhaite obtenir sur le film. Pour déterminer cette lumination, la première étape est de réaliser un sensitogramme du type de film utilisé, ensuite il faut réaliser un cliché dans des conditions radiologiques déterminées avec un étalon d'épaisseur connue.

Ces conditions radiologiques déterminées sont, par exemple,
- une densité optique de référence $DO_{refo}$ choisie en fonction des habitudes du praticien, par exemple $DO_{refo} = 1$
- un étalon d'épaisseur $E_o$,
- une tension d'alimentation $V_o$,
- une valeur du temps de pose $t_o$,
- une valeur du produit $I_o \times t_o$,

Pour ce cliché, on mesure la densité optique $DO_m$ ainsi que les valeurs $M_o$, $I_o$, $t_o$, ce qui permet de calculer l'épaisseur équivalente $E_p$ à l'aide de la formule (7). On calcule ensuite le rendement $D_f$ sur le film à l'aide de la formule (6), ce qui permet de calculer la lumination reçue par le film $L_{film}$ par la formule :

$$L_{film} \ = \ D_f \times I_o \times t_o \quad (23)$$

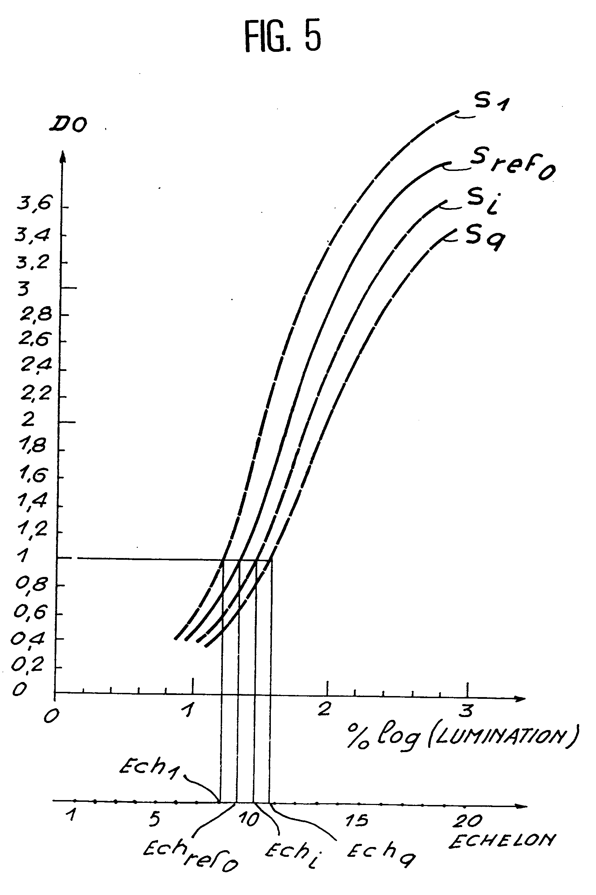

La densité optique de référence $DO_{refo}$ permet de calculer l'échelon d'éclairement correspondant à $DO_{refo}$ sur la courbe sensitométrique du film utilisé (figure 5), cette courbe ayant été tracée à l'aide d'un sensitographe et d'un densitomètre. Ceci permet de tenir compte des caractéristiques de la machine à développer qui est utilisée. La courbe est enregistrée, par exemple, sous la forme d'une fonction dans le microprocesseur 19 (fig.1).

La densité optique mesurée $DO_m$ permet de calculer l'échelon de mesure $Ech_m$ qui est la valeur de l'échelon d'éclairement correspondant à $DO_m$ sur la courbe sensitométrique (fig. 5).

Avec les valeurs $L_{film}$ de la lumination sur le film, de l'échelon de référence $Ech_{ref}$ et de l'échelon de mesure $Ech_m$, il est possible de calculer la lumination de référence $L_{ref}$ pour obtenir la densité optique $DO_{refo}$ en utilisant l'équation qui définit le changement d'échelle entre la lumination et l'échelon d'éclairement de l'axe des abscisses de la courbe sensitométrique (figure 5), soit :

$$Ech_m \ = \ Ech_{ref} \ + \ K.\log_{10} \left[ \frac{L_{film}}{L_{ref}} \right] \qquad (24)$$

De cette équation (24), on tire :

$$L_{ref} \ = \ L_{film} \times \exp \left[ \log_{10} \left[ \frac{Ech_{ref} \ - \ Ech_m}{K} \right] \right] \qquad (25)$$

avec

$$K = 2/\log_{10}(2) \quad (26)$$

La constante sensitométrique K correspond à l'échelle retenue pour les échelons d'éclairement. La valeur $L_{ref}$ dépend de $t_o$ à travers $L_{film}$ par les équations (23) et (25). Ainsi, la valeur $L_{ref}$ est sensible aux effets de non réciprocité du film. Pour corriger l'influence de la non réciprocité sur la valeur de $L_{ref}$, il suffit d'utiliser dans l'équation (23), la valeur $L_{film}$ définie par :

$$L'_{film} = \frac{D_f \times I_o \times t_o}{CNRT(t_o)} \quad (23')$$

Cette lumination de référence $L_{ref}$ est celle qui doit être utilisée dans l'équation (10) pour obtenir la densité optique de référence $DO_{refo}$ et la formule (25) montre qu'elle dépend, notamment, de la différence entre l'échelon de référence et l'échelon de mesure.

La connaissance de la lumination reçue par le film permet de connaître $d_i$ par application de la formule (22) et à en déduire $CNRD(d_i)$ par la formule (20).

Pour une densité optique du film radiographique autre que $DO_{refo} = 1$, il est nécessaire d'effectuer de nouveau les opérations décrites ci-dessus de manière à déterminer les nouvelles valeurs de $CNRT(t_i)$ et de $L_{ref}$.

Afin de simplifier ces opérations, les coefficients $CNRT(t_i)$ peuvent être obtenus en effectuant les opérations suivantes :

(g1) réalisation à l'aide d'un sensitographe à temps variable, d'un premier sensitogramme $S_{refo}$ (figure 5) lorsque le temps de pose est réglé pour un temps de référence $t_{refo}$;

(g2) réalisation à l'aide du même sensitographe à temps variable, de q sensitogrammes $S_1$ à $S_q$ (figure 5) pour q temps de pose différents $t_i$;

(g3) choix d'une densité optique de référence $DO_{refo}$, par exemple $DO_{refo} = 1$

(g4) mesure sur chaque sensitogramme, de l'échelon d'éclairement $Ech_{refo}$, $Ech_1...Ech_i...Ech_q$ correspondant à la densité optique $DO_{refo} = 1$

(g5) calcul du coefficient $CNRT(t_i)$ par l'équation :

$$CNRT(t_i) = \exp\left[\log_{10}\left[\frac{Ech_{refo} - Ech_i}{K}\right]\right] \quad (28)$$

Si le praticien décide de travailler à une densité optique différente, il est proposé, afin d'éviter l'étalonnage décrit ci-dessus, d'utiliser la densité optique corrigée volontairement pour le noircissement $DO_{cvn}$. Alors la lumination de référence $L_{ref}$, utilisée dans l'équation (10) doit être remplacée par la lumination corrigée $L_{cvn}$ qui s'exprime par :

$$L_{cvn} = L_{ref} \times \exp[CVN/\Gamma \times P \times \log(10)] \quad (27)$$

où

- CVN est la correction volontaire de noircissement exprimée par un nombre entier de -10 à + 10 par exemple,
- P est le pas en densité optique, par exemple 0,1,
- $\Gamma$ est la pente de la partie linéaire de la courbe sensitométrique (figure 5).

Le procédé qui vient d'être décrit montre que sa mise en oeuvre nécessite un certain nombre d'étalonnages qui sont, en résumé, les suivants :

(a) l'étalonnage du système radiologique de manière à déterminer les modèles analytiques

$$D_{se} = f'(V_m, E_p) \quad (4)$$

avec cassette sans écran et

$$D_c = f''(V_m, E_p) \quad (6)$$
$$E_p = g''(V_m, D_c) \quad (7)$$

avec cassette et écran ;

la différence $D_f = (D_{se} - D_c)$ (équation (8)) permettra de déduire le rendement absorbé par l'écran;

(b) l'étalonnage du film de manière à déterminer la loi de non réciprocité $CNRT(t)$ exprimée en fonction du temps; cette loi sera utilisée pour déterminer la loi de non réciprocité $CNRD(d)$ exprimée en fonction du débit;

(c) l'étalonnage de la lumination de référence $L_{ref}$.

Ces différents étalonnages ayant été effectués, les différentes opérations proprement dites du procédé sont les suivantes :

(d) choix, par le praticien, de la valeur de noircissement ou de la valeur de la correction volontaire de noircissement de manière à déterminer la lumination cible $L_{cvn}$ que doit recevoir le film dans des conditions de référence fixées pour arriver au noircissement (ou densité optique) choisi. La lumination $L_{cvn}$ est calculée à partir de l'équation (27) où la lumination $L_{ref}$ est déterminée par l'étalonnage (c) et les équations (25) et (26);

(e1) mise en place de l'objet à radiographier,

(e2) déclenchement du début de la pose par le praticien;

(e3) mesure après un temps t' du rendement $D_{c1}$ au niveau de la cellule (12);

(e4) mesure de l'épaisseur équivalente $E_1$ par l'équation (7);

(e5) calcul du rendement $D_{f1}$ au niveau du film pour l'épaisseur $E_1$ par l'équation (8);

(e6) calcul de la lumination $L_f$ reçue par le film par l'équation :
$$L_f = L_{am} + D_{f1} \times \delta mA.s/CNRD \text{ (débit - film)} \quad (9')$$

(e7) calcul de la lumination $L_{ra}$ restant à acquérir pour obtenir le noircissement (ou densité optique) choisi par l'équation
$$L_{ra} = L_{cvn} - L_f \quad (10')$$

(e8) calcul prévisionnel des mA.s restant à débiter $mAs_{ra}$ pour obtenir le noircissement (ou densité optique) par l'équation :
$$mAs_{ra} = L_{ra}/D_{f1} \times CNRD \text{ (débit - film)} \quad (11')$$

(e9) mesure des mA.s débités depuis le début de l'opération (e3) ;

(e10)

    - arrêt de la pose lorsque les mA.s mesurés en (e9) sont égaux ou supérieurs à $mAs_{ra}$.

    - ou retour à l'opération (e3) lorsque les mA.s mesurés en (e9) sont inférieures à $mAs_{ra}$.

La description du procédé qui vient d'être faite correspond à une certaine configuration du système de radiologie. Dans le cas où ce système peut prendre plusieurs configurations tels que, par exemple, le choix

- du matériau d'anode,
- des dimensions du foyer,
- du filtre de modification du spectre,
- de la collimation,
- de la présence ou absence d'une grille anti-diffusante,
- du type de récepteur image,
- du type de cellule de détection,

il est nécessaire d'effectuer pour chacune de ces configurations des étalonnages (a), (b) et (c). Le nombre de ces étalonnages peut être réduit en tenant compte des similitudes de comportement d'une configuration à l'autre comme cela a été décrit pour l'étalonnage (a) dans la demande de brevet n° 89 07686 déposée le 9 juin 1989 et correspondant à EP-A-0 402 244.

Lors de la mise en oeuvre du procédé par le praticien, ce dernier définit la configuration dont les caractéristiques sont transmises au microprocesseur (19) de manière que ce dernier utilise les modèles correspondants.

Le procédé selon l'invention a été décrit dans son application à un récepteur 17 du type couple film-écran.

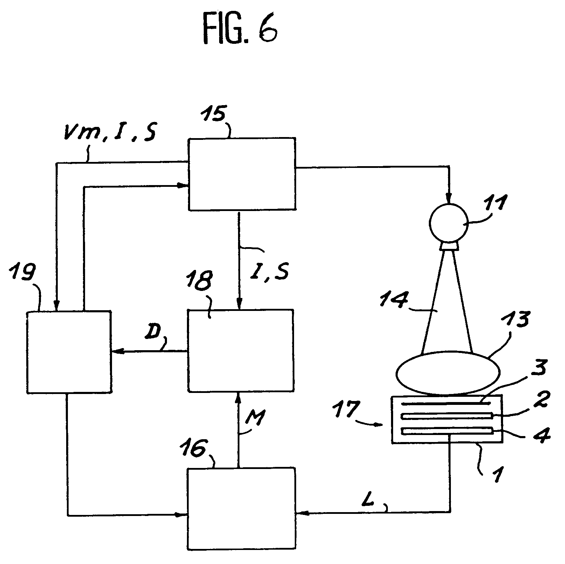

Le procédé selon l'invention a été décrit dans une application à un système de radiologie (figure 1) dans lequel la cellule de détection 12 des rayons X est placée à l'extérieur du récepteur 17 mais ledit procédé est applicable à un système de radiologie (figure 6) dans lequel la cellule de détection est incorporée dans le récepteur sous l'écran 2 (référence 4) et est sensible au rayonnement lumineux émis par ledit écran. Un tel récepteur muni d'une telle cellule de détection a été décrit dans la demande de brevet français n° 89 05668 (FR-A-2 646 515) déposée le 28 avril 1989 et intitulée Cassette de radiologie avec cellule détectrice d'exposeur automatique incorporée.

Avec ce nouveau type de récepteur comportant une cellule de détection 4 des rayons lumineux émis par l'écran 2, il n'est plus nécessaire d'effectuer les opérations de calibration (a) et (b) du procédé qui avaient pour but de tenir compte de l'atténuation du rayonnement X introduite par le film et l'écran.

En outre, par voie de conséquence, les opérations (e4) et (e5) ne sont plus nécessaires.

Ceci induit à un procédé modifié qui comporte les opérations suivantes :

(a'') un étalonnage pour déterminer la lumination de référence $L_{ref}$ que doit recevoir le film, dans des conditions de référence fixées, pour arriver au noircissement (ou densité optique) choisi comme valeur de référence par le praticien ;

(b1) la mise en place de l'objet à radiographier suivie des opérations suivantes :

(b2) déclenchement au début de la pose par le praticien ;

(b3) mesure de rendement $D_{f1}$ un certain temps t' après le début de la pose ;

(b4) calcul de la lumination $L_f$ reçue par le film selon l'équation :
$$L_f = L_{am} + D_{f1} \times \delta mA.s \quad (9)$$

(b5) calcul de la lumination $L_{ra}$ restant à acquérir pour obtenir le noircissement (ou densité optique) choisi par l'équation :

$$L_{ra} = L_{ref} - L_f \quad (10)$$

(b6) calcul prévisionnel des mA.s restant à débiter $mAs_r$ pour obtenir le noircissement (ou densité optique) choisi par l'équation :

$$mAs_r = L_{ra}/D_{fl} \quad (11)$$

(b7) mesure des mA.s débités $mAs_{mes}$ depuis le début de l'opération (b3) ;

(b8)
- arrêt de la pose lorsque les mA.s mesurés $mAs_{mes}$ en (b7) sont égaux ou supérieurs à $mAs_r$,
- ou retour à l'opération (b3) lorsque les mA.s mesurés en (b7) sont inférieurs à $mAs_r$.

Il est à noter que les particularités et modifications qui ont été décrites pour le procédé utilisant la cellule de détection 12 du rayonnement X s'appliquent à celui utilisant la cellule de détection 4 du rayonnement lumineux dans la mesure où elles concernent l'opération de calibrage (a″) et les opérations (b1) et (b8).

**Revendications**

1. Procédé de détermination automatique de la durée d'exposition d'un film radiographique dans un système de radiologie prévu pour examiner un objet (13) qui comprend un tube (11) à rayons X dont la tension d'alimentation peut prendre diverses valeurs $V_m$, à variation continue ou discrète, et qui émet un faisceau (14) de rayons X sous forme d'impulsions de durée variable S en direction de l'objet (13) à examiner, un récepteur (17) du rayonnement X ayant traversé l'objet (13) pour réaliser une image dudit objet, ledit récepteur étant composé d'au moins un écran renforçateur (2) et d'un film (3) sensible à la lumière émise par cet écran, une cellule de détection (4) qui est prévue pour détecter les rayons lumineux émis par ledit écran (2), placée dans ledit récepteur en aval dudit écran, et qui permet de convertir une grandeur physique caractérisant le faisceau de rayons X en un signal de mesure L, un circuit intégrateur (16) qui intègre le signal de mesure L pendant la durée S et fournit un signal M et un dispositif de calcul (18) du rendement D donné par le rapport de M par le produit du courant anodique I du tube par la durée de la pose S, I x S (ou mA.s), le procédé comprenant les opérations suivantes :

   (a″) un étalonnage pour déterminer la lumination de référence $L_{ref}$ que doit recevoir le film, dans des conditions de référence fixées, pour arriver au noircissement (ou densité optique) choisi comme valeur de référence par le praticien;

   (b1) mise en place de l'objet (13) à radiographier ;

   (b2) déclenchement du début de la pose par le praticien;

   (b3) mesure du rendement $D_{fl}$ un certain t′ après le début de la pose ou, suivant le cas, depuis la dernière mesure;

   (b4) calcul de la lumination $L_f$ reçue par le film selon l'équation :

   $$L_f = L_{am} + D_{f1} \times \delta mA.s \quad (9)$$

   dans laquelle $L_{am}$ est la lumination reçue par le film avant l'opération (b3) et $\delta mA.s$ est le nombre de mA.s débités dans le temps pendant le temps t′ et est défini par le produit du courant I du tube par le temps d'intégration t′;

   (b5) calcul de la lumination $L_{ra}$ restant à acquérir pour obtenir le noircissement (ou densité optique) choisi par l'équation :

   $$L_{ra} = L_{ref} - L_f \quad (10)$$

   (b6) calcul prévisionnel des mA.s restant à débiter ($mAs_r$) pour obtenir le noircissement (ou densité optique) choisi par l'équation :

   $$mAs_r = L_{ra}/D_{f1} \quad (11)$$

   (b7) mesure des mA.s ($mAs_{mes}$) depuis le début de la pose ou, suivant le cas, de la mesure précédente;

   (b8) arrêt de la pose lorsque la mesure $mAs_{mes}$ devient égale ou supérieure à

   $$mAs_r = L_{ra}/D_{f1} \quad (11)$$

   - ou retour à l'opération (b3) lorsque la mesure $mAs_{mes}$ en (b7) est inférieure à $mAs_r$ .

2. Procédé de détermination automatique de la durée d'exposition d'un film radiographique dans un système de radiologie prévu pour examiner un objet (13) qui comprend un tube (11) à rayons X dont la tension d'alimentation V peut prendre diverses valeurs $V_m$, à variation continue ou discrète, et qui émet un faisceau (14) de rayons X sous forme d'impulsions de durée variable S en direction de l'objet (13) à examiner, un récepteur (17) du rayonnement X ayant traversé l'objet (13) pour réaliser une image dudit objet, ledit récepteur étant composé d'au moins un écran renforçateur et d'un film sensible à la lumière émise par cet écran, une cellule de détection (12) des rayons X ayant traversé l'objet à examiner, placée derrière le récepteur image (17) qui permet de convertir une grandeur physique caractérisant le faisceau (14) de rayons X en un signal de mesure L, un circuit intégrateur (16) qui intègre le signal de mesure L pendant la durée S et fournit un signal M et un dispositif de calcul (18) du rendement D donné par le rapport de M par le produit I x S (ou mA.s) du courant anodique I du tube par la durée de la pose S, le procédé comprenant les étalonnages suivants :

(a) un premier étalonnage du système de radiologie à l'aide d'objets d'épaisseur $E_p$ en utilisant un récepteur sans le ou les écrans renforçateurs de manière à déterminer la fonction de rendement $D_{se}$ telle que :

$$D_{se} = f' (V_m, E_p) \quad (4)$$

et la fonction inverse

$$E_p = g' (V_m, D_{se}) \quad (5)$$

(b) un deuxième étalonnage du système de radiologie à l'aide des objets d'épaisseur $E_p$ en utilisant ledit récepteur (17) avec le ou les écrans renforçateurs de manière à déterminer la fonction de rendement $D_c$ telle que :

$$D_c = f'' (V_m, E_p) \quad (6)$$

et la fonction inverse :

$$E_p = g'' (V_m, D_c) \quad (7)$$

et le calcul de la fonction de rendement $D_f$ telle que :

$$D_f = f' (V_m, E_p) - f'' (V_m, E_p) \quad (8)$$

dans laquelle f' et f'' sont des fonctions qui décrivent le comportement du rendement D en fonction des paramètres $V_m$ et $E_p$ pour les configurations données du système;

(c) un troisième étalonnage pour déterminer la lumination de référence $L_{ref}$ que doit recevoir le film, dans des conditions de référence fixées, pour arriver au noircissement (ou densité optique) choisi comme valeur de référence par le praticien; puis les opérations suivantes :

(e1) mise en place de l'objet (13) à radiographier;

(e2) déclenchement du début de la pose par le praticien;

(e3) mesure du rendement $D_{c1}$ un certain t' après le début de la pose ou, suivant le cas, depuis la dernière mesure;

(e4) mesure de l'épaisseur équivalente $E_1$ par l'équation (7);

(e5) calcul du rendement $D_{f1}$ au niveau du film pour l'épaisseur $E_1$ par l'équation (8);

(e6) calcul de la lumination $L_f$ reçue par le film selon l'équation :

$$L_f = L_{am} + D_{f1} \times \delta mA.s \quad (9)$$

dans laquelle $L_{am}$ est la lumination reçue par le film avant l'opération (e3) et $\delta mA.s$ est le nombre de mA.s débités dans le temps pendant le temps t' et est défini par le produit du courant I du tube par le temps d'intégration t';

(e7) calcul de la lumination $L_{ra}$ restant à acquérir pour obtenir le noircissement (ou densité optique) choisi par l'équation :

$$L_{ra} = L_{ref} - L_f \quad (10)$$

(e8) calcul prévisionnel des mA.s restant à débiter $mAs_{ra}$ pour obtenir le noircissement (ou densité optique) choisi par l'équation

$$mAs_r = L_{ra}/D_{f1} \quad (11)$$

(e9) mesure des mA.s débités $mAs_{mes}$ depuis le début de l'opération (e3) ou, suivant le cas, de la mesure précédente;

(e10)
- arrêt de la pose lorsque les mA.s mesurés $mAs_{mes}$ en (e9) sont égaux ou supérieurs à $mAs_r$
- ou retour à l'opération (e3) lorsque les mA.s mesurés en (e9) sont inférieurs à $mAs_r$.

3. Procédé selon la revendication 1 ou la revendication 2, caractérisé en ce qu'il comprend en outre
- une opération de conversion de $mAs_{ra}$ en une valeur $CE_{cible}$ dans les unités de la cellule (4) telle que :

$$CE_{cible} = mAs_r \times D_{f1}$$

et
- une opération de décrémentation de la valeur cible $CE_{cible}$ par les signaux reçus de la cellule de détection (12;4) de manière à arrêter la pose lorsque la valeur décrémentée devient inférieure ou égale à une valeur prédéterminée $Val_0$.

4. Procédé selon la revendication 1, caractérisé en ce que l'opération (b6) comprend, en outre, une opération de calcul des mA.s débités ($mAs_c$) pendant les opérations de calcul (b4) à (b6) de durée $t_c$ par l'équation :

$$mAs_c = I \times t_c \quad (13)$$

de manière à déterminer la valeur réelle des mA.s restant à acquérir ($mAs_{ra}$) telle que :

$$mAs_{ra} = mAs_r - mAs_c \quad (12)$$

5. Procédé selon la revendication 4, caractérisé en ce que l'opération de calcul de $mAs_r$ est renouvelée périodiquement pendant la pose à des instants $t_1$, $t_2$...$t_n$ séparés par une durée qui est au moins égale au temps de calcul $t_c$ des opérations (b4) à (b6).

6. Procédé selon la revendication 1, caractérisé en ce que des calculs corrigés de $L_f$ et de $L_{ra}$ sont effectués respectivement suivant les formules suivantes :

$$L''_f = L_{am} + D_{f1} \times \delta mA.s/CNRD \text{ (débit - film)} \quad (9')$$

et

$$mAs''_{ra} = \frac{L_{ra}}{D_{f1}} \times CNRD \text{ (débit - film)} \quad (11')$$

formules dans lesquelles CNRD (débit-film) est le coefficient de non réciprocité indexé en débit-film du récepteur (17) tel que

$$\text{débit - film} = D_{f1} \times I \quad (17)$$

7. Procédé selon la revendication 6, caractérisé en ce que le coefficient CNRD (débit-film) est obtenu en effectuant les opérations suivantes :
   - mesure des coefficients de non réciprocité CNRT ($t_i$) du couple film-écran en fonction des temps de pose $t_i$,
   - mesure pour chaque temps de pose ($t_i$) du débit-film $d_i$,
   - détermination de la fonction de modélisation des coefficients CNRD ($d_i$) telle que :
   $$CNRD (d) = A'_0 + A'_1 \log 1/d + A'_2 [\log 1/d]^2 \quad (20)$$
   $A'_0$, $A'_1$ et $A'_2$ étant des paramètres qui sont estimés à partir des points de mesure, ce qui permet de déterminer le coefficient correspondant à un débit-film donné.

8. Procédé selon la revendication 7, caractérisé en ce que le débit-film d est mesuré par la cellule (4,12).

9. Procédé selon la revendication 8, caractérisé en ce que le débit-film $d_i$ est donné par la formule :

$$d_i = \frac{L_{ref} \times CNRT (t_i)}{t_i} \quad (28)$$

10. Procédé selon la revendication 9, caractérisé en ce que les coefficients CNRT ($t_i$) en fonction du temps de pose $t_i$ sont obtenus en effectuant les opérations suivantes.
    ($a_1$) la modification du courant de chauffage du tube (11) de manière à obtenir différentes valeurs dudit courant,
    ($a_2$) le relevé des valeurs M ($t_i$) fournies par le circuit intégrateur (16) pour différents temps de pose ($t_i$) de manière à obtenir une densité optique $DO_1$ du film
    ($a_3$) le calcul du rapport

    $$\frac{M (t_i)}{M (t_{ref})} \quad (29)$$

    qui donne le coefficient CNRT ($t_i$) avec M ($t_{ref}$) la valeur M ($t_i$) pour $t_i = t_{ref}$.

11. Procédé selon la revendication 9, caractérisé en ce que les coefficients CNRT ($t_i$) sont obtenus en effectuant les opérations suivantes :
    (g1) réalisation à l'aide d'un sensitographe à temps variable, d'un premier sensitogramme $S_{refo}$ lorsque le temps de pose est réglé pour un temps de référence $t_{refo}$;
    (g2) réalisation à l'aide du même sensitographe à temps variable, de q sensitogrammes $S_1$ à $S_q$ pour q temps de pose différents $t_i$;
    (g3) choix d'une densité optique de référence $DO_{refo}$,
    (g4) mesure sur chaque sensitogramme, de l'échelon d'éclairement Echrefo, $Ech_1$...$Ech_i$...$Ech_q$ correspondant à la densité optique $DO_{refo}$,
    (g5) calcul du coefficient CNRT ($t_i$) par l'équation :

$$CNRT\ (t_i) = \exp\left[\log_{10}\left[\frac{Ech_{refo} - Ech_i}{K}\right]\right] \qquad (28)$$

avec $K = 2/\log_{10}(2)$

**12.** Procédé selon la revendication 10 ou 11 caractérisé en ce qu'il comprend, en outre, une opération de modélisation des coefficients CNRT $(t_i)$ sous la forme d'un modèle analytique :

$$CNRT\ (t) = Ao + A_1 \log t + A_2 [\log t]^2 \quad (18)$$

$A_0$, $A_1$ et $A_2$ étant des paramètres qui sont estimés à partir des points de mesure.

**13.** Procédé selon l'une quelconque des revendications 1 à 12, caractérisé en ce que l'opération (a") d'étalonnage de la lumination de référence comprend les opérations suivantes :
  - la réalisation d'un cliché dans des conditions radiologiques déterminées pour une densité optique de référence, par exemple $DO_{refo} = 1$, un étalon d'épaisseur $E_o$, une tension d'alimentation $V_o$ et une valeur du produit d'un courant anodique $I_o$ du tube par une valeur du temps de pose $t_o$;
  - calcul du rendement $D_{fo}$ par le dispositif (18);
  - calcul de la luminance $L_{film}$ sur le film par la formule :

$$L_{film} = D_{fo} \times I_o \times t_o \quad (23)$$

  - calcul de l'échelon d'éclairement $Ech_{ref}$ correspondant à la densité optique de référence $DO_{refo}$ à l'aide de la courbe sensitométrique;
  - mesure de la densité optique $DO_m$ du cliché obtenu et calcul de l'échelon d'éclairement $Ech_m$ à l'aide de la courbe sensitométrique, et
  - calcul de la lumination de référence $L_{ref}$ par la formule :

$$L_{ref} = L_{film} \times \exp\left[\log_{10}\left[\frac{Ech_{ref} - Ech_m}{K}\right]\right] \qquad (25)$$

avec

$$K = 2/\log_{10}(2) \quad (26)$$

**14.** Procédé selon les revendications 12 et 13, caractérisé en ce que la luminance corrigée sur le film $L'_{film}$ est calculée par la formule :

$$L'_{film} = \frac{D_{fo} \times I_o \times t_o}{CNRT\ (t_o)} \quad (23')$$

**15.** Procédé selon la revendication 13 ou 14, caractérisé en ce qu'une lumination corrigée $L_{cvn}$ est calculée selon la formule :

$$L_{cvn} = L_{ref} \times \exp\left[CVN/\Gamma \times P \times \log(10)\right] \quad (27)$$

de manière à obtenir un noircissement (ou densité optique) différent, formule dans laquelle :
  - CVN est la correction volontaire de noircissement exprimée par un nombre entier par exemple,
  - P est le pas en densité optique,
  - $\Gamma$ est la pente de la partie linéaire de la courbe sensitométrique.

## Patentansprüche

**1.** Verfahren zur automatischen Bestimmung der Belichtungsdauer eines Röntgenfilms in einem zur Untersuchung eines Gegenstandes (13) vorgesehenen radiologischen System, das versehen ist mit einer Röntgenröhre (11), deren Versorgungsspannung verschiedene kontinuierlich oder diskret veränderliche Werte $V_m$ annehmen kann und die ein Bündel (14) von Röntgenstrahlen in Form von Impulsen veränderlicher Dauer S in Richtung des zu untersuchenden Gegenstandes (13) aussenden kann, einem Empfänger (17) für die Röntgenstrahlung, die den Gegenstand (13) durchquert hat, um ein Bild des Gegenstandes zu verwirklichen, wobei der Empfänger aus wenigstens einem Verstärkerschirm (2) und einem für das von diesem Schirm ausgesandte Licht empfindlichen Film (3) aufgebaut ist, einer Detektorzelle (4), die dazu vorgesehen ist, die von dem Schirm (2) ausgesandten Lichtstrahlen zu erfassen, im Empfänger hinter dem Schirm angeordnet ist und eine das Bündel von Röntgenstrahlen kennzeichnende physikalische Größe in ein Meßsignal L umwandeln kann, einer Integratorschaltung (18), die das Meßsignal L während der Dauer S integriert und ein Signal M ausgibt, und einer Einrichtung (18) zur Berechnung des Wirkungsgrades D, der durch das Verhältnis von M zum Produkt des Anodenstroms I der Röhre mit der Aufnahmedauer S, $I \times S$ (oder $mA \cdot s$), gegeben ist, wobei das Verfahren die folgenden Operationen enthält:

(a") eine Eichung unter festen Referenzbedingungen für die Bestimmung der Referenzbelichtung $L_{ref}$, die der Film empfangen muß, um eine vom Anwender als Referenzwert gewählte Schwärzung (oder optische Dichte) zu erreichen;

(b1) Anordnen des der röntgenologischen Untersuchung zu unterziehenden Gegenstandes (13);

(b2) Auslösen des Beginns der Aufnahme durch den Anwender;

(b3) Messen des Wirkungsgrades $D_{f1}$ ein bestimmtes t' nach dem Beginn der Aufnahme oder je nach Fall ab der letzten Messung;

(b4) Berechnen der vom Film empfangenen Belichtung $L_f$ gemäß der Gleichung:

$$L_f = L_{am} + D_{f1} \times \delta mA \cdot s, \quad (9)$$

in der $L_{am}$ die vom Film vor der Operation (b3) empfangene Belichtung ist und $\delta mA \cdot s$ die Anzahl der pro Zeiteinheit während der Zeit t' gelieferten $mA \cdot s$ ist und durch das Produkt des Stroms I der Röhre mit der Integrationszeit t' definiert ist;

(b5) Berechnen der noch zu erlangenden Belichtung $L_{ra}$, um die gewählte Schwärzung (oder optische Dichte) zu erhalten, durch die Gleichung:

$$L_{ra} = L_{ref} - L_f \quad (10)$$

(b6) prognostisches Berechnen der noch zu liefernden $mA \cdot s$ ($mAs_r$), um die gewählte Schwärzung (oder optische Dichte) zu erhalten, durch die Gleichung:

$$mAs_r = \frac{L_{ra}}{D_{f1}} \quad (11)$$

(b7) Messen der $mA \cdot s$ ($mAs_{mes}$) ab dem Beginn der Aufnahme oder je nach Fall ab der vorangehenden Messung;

(b8) Beenden der Aufnahme, wenn die Messung $mAs_{mes}$ gleich oder größer wird als

$$mAs_r = \frac{L_{ra}}{D_{f1}} \quad (11)$$

- oder Rückkehr zur Operation (b3), wenn die Messung $mAs_{mes}$ in (b7) kleiner als $mAs_r$ ist.

2. Verfahren zur automatischen Bestimmung der Belichtungsdauer eines Röntgenfilms in einem zur Untersuchung eines Gegenstandes (13) vorgesehenen radiologischen System, das versehen ist mit einer Röntgenröhre (11), deren Versorgungsspannung V verschiedene kontinuierlich oder diskret veränderliche Werte $V_m$ annehmen kann und die ein Bündel (14) von Röntgenstrahlen in Form von Impulsen mit veränderlicher Dauer S in Richtung des zu untersuchenden Gegenstandes (13) aussendet, einem Empfänger (17) für die Röntgenstrahlung, die den Gegenstand (13) durchquert hat, um ein Bild des Gegenstandes zu verwirklichen, wobei der Empfänger aus wenigstens einem Verstärkerschirm und einem für das von diesem Schirm ausgesandte Licht empfindlichen Film aufgebaut ist, einer Detektorzelle (12) für die Röntgenstrahlen, die den zu untersuchenden Gegenstand durchquert haben, die hinter dem Bildempfänger (17) angeordnet ist und die eine das Bündel (14) von Röntgenstrahlen X kennzeichnende physikalische Größe in ein Meßsignal L umwandeln kann, einer Integratorschaltung (16), die das Meßsignal L während der Dauer S integriert und ein Signal M ausgibt, und einer Einrichtung (18) zur Berechnung des Wirkungsgrades D, der durch das Verhältnis von M zum Produkt $I \times S$ (oder $mA \cdot s$) des Anodenstroms I der Röhre mit der Aufnahmedauer S gegeben ist, wobei das Verfahren die folgenden Eichungen enthält:

a) eine erste Eichung des radiologischen Systems mit Hilfe von Gegenständen der Dicke $E_p$, indem ein Empfänger ohne den oder die Verstärkerschirme in der Weise verwendet wird, daß die Funktion des Wirkungsgrades $D_{se}$:

$$D_{se} = f' (V_m, E_p) \quad (4)$$

sowie die inverse Funktion

$$E_p = g' (V_m, D_{se}) \quad (5)$$

bestimmt werden;

(b) eine zweite Eichung des radiologischen Systems mit Hilfe der Gegenstände des Dicke $E_p$, indem der Empfänger (17) mit dem oder den Verstärkerschirmen in der Weise verwendet wird, daß die Funktion des Wirkungsgrades $D_c$:

$$D_c = f''(V_m, E_p) \quad (6)$$

sowie die inverse Funktion:

$$E_p = g''(V_m, D_c) \quad (7)$$

bestimmt werden,

und die Berechnung der Funktion des Wirkungsgrades $D_f$, derart, daß:

$$D_f = f'(V_m, E_p) - f''(V_m, E_p), \quad (8)$$

wobei $f'$ und $f''$ Funktionen sind, die das Verhalten des Wirkungsgrades $D$ in Abhängigkeit von den Parametern $V_m$ und $E_p$ für die gegebenen Konfigurationen des Systems beschreiben;

(c) eine dritte Eichung unter festen Referenzbedingungen zur Bestimmung der Referenzbelichtung $L_{ref}$, die der Film empfangen muß, um die Schwärzung (oder optische Dichte) zu erzielen, die vom Anwender als Referenzwert gewählt worden ist; dann die folgenden Operationen:

(e1) Anordnen des der röntgenologischen Untersuchung zu unterziehenden Gegenstandes (13);

(e2) Auslösen des Beginns der Aufnahme durch den Anwender;

(e3) Messen des Wirkungsgrades $D_{c1}$ eine bestimmte Zeit $t'$ nach dem Beginn der Aufnahme oder je nach Fall ab der letzten Messung;

(e4) Messen der äquivalenten Dicke $E_1$ durch die Gleichung (7);

(e5) Berechnen des Wirkungsgrades $D_{f1}$ auf Höhe des Films für die Dicke $E_1$ durch die Gleichung (8);

(e6) Berechnen der vom Film empfangenen Belichtung $L_f$ gemäß der Gleichung:

$$L_f = L_{am} + D_{f1} \times \delta mA \cdot s, \quad (9)$$

in der $L_{am}$ die vom Film vor der Operation (e3) empfangene Belichtung ist und $\delta mA \cdot s$ die Anzahl der pro Zeiteinheit während der Zeit $t'$ gelieferten $mA \cdot s$ ist und durch das Produkt des Stroms $I$ der Röhre mit der Integrationszeit $t'$ definiert ist;

(e7) Berechnen der noch zu erlangenden Belichtung $L_{ra}$, um die gewählte Schwärzung (oder optische Dichte) zu erhalten, durch die Gleichung:

$$L_{ra} = L_{ref} - L_f \quad (10)$$

(e8) prognostisches Berechnen der noch zu liefernden $mA \cdot s$ ($mAs_r$), um die gewünschte Schwärzung (oder optische Dichte) zu erhalten, durch die Gleichung

$$mAs_r = \frac{L_{ra}}{D_{f1}} \quad (11)$$

(e9) Messen der $mA \cdot s$, die ab dem Beginn der Operation (e3) oder je nach Fall ab der vorangehenden Messung geliefert worden sind, $mAs_{mes}$;

(e10)

- Beenden der Aufnahme, wenn die $mA \cdot s$, die in (e9) gemessen werden, $mAs_{mes}$, gleich oder größer sind als $mAs_r$;
- oder Rückkehr zur Operation (e3), wenn die in (e9) gemessenen $mA \cdot s$ kleiner als $mAs_r$ sind.

3. Verfahren gemäß Anspruch 1 oder Anspruch 2, dadurch gekennzeichnet, daß es außerdem enthält:

- eine Operation der Umwandlung von $mAs_{ra}$ in einen Wert $CE_{Target}$ in den Einheiten der Zelle (4), derart, daß:

$$CE_{Target} = mAs_r \times D_{f1}$$

und

- eine Operation der Dekrementierung des Target-Wertes $CE_{Target}$ durch die von der Detektorzelle (12; 4) empfangenen Signale, so daß die Aufnahme beendet wird, wenn der dekrementierte Wert kleiner oder gleich einem vorgegebenen Wert $Val_0$ wird.

4. Verfahren gemäß Anspruch 1, dadurch gekennzeichnet, daß die Operation (b6) außerdem eine Operation des Berechnens der $mA \cdot s$, die während der Rechenoperationen (b4) bis (b6) der Dauer $t_c$ geliefert werden ($mAs_c$), durch die Gleichung:

$$mAs_c = I \times t_c \quad (13)$$

enthält, so daß der wirkliche Wert der $mA \cdot s$ bestimmt wird, der noch zu erreichen ist ($mAs_{ra}$), derart, daß:

$$mAs_{ra} = mAs_r - mAs_c. \quad (12)$$

5. Verfahren gemäß Anspruch 4, dadurch gekennzeichnet, daß die Operation der Berechnung von $mAs_r$ während der Aufnahme in Zeitpunkten $t_1, t_2, ..., t_n$, die durch eine Dauer voneinander getrennt sind, die wenigstens gleich der Rechenzeit $t_c$ der Operationen (b4) bis (b6) ist, periodisch neu ausgeführt wird.

6. Verfahren gemäß Anspruch 1, dadurch gekennzeichnet, daß korrigierte Berechnungen von $L_f$ bzw. von $L_{ra}$ gemäß den folgenden Formeln ausgeführt werden:

$$L''_f = L_{am} + D_{f1} \times \frac{\delta mA \cdot s}{CNRD(Filmrate)} \quad (9')$$

bzw.

$$mAs''_{ra} = \frac{L_{ra}}{D_{f1}} \times CNRD(Filmrate) \quad (11')$$

wobei in den Formeln CNRD(Filmrate) der NichtreziprozitätsKoeffizient ist, der durch die Filmrate des Empfängers (17) indexiert ist, derart, daß

$$Filmrate = D_{f1} \times I. \quad (17)$$

7. Verfahren gemäß Anspruch 6, dadurch gekennzeichnet, daß der CNRD(Filmrate)-Koeffizient dadurch erhalten wird, daß die folgenden Operationen ausgeführt werden:
   - Messen der Nichtreziprozitäts-Koeffizienten $CNRT(t_i)$ des Paars Film-Schirm in Abhängigkeit von der Aufnahmezeit $t_i$,
   - Messen der Filmrate $d_i$ für jede Aufnahmezeit $(t_i)$,
   - Bestimmen der Modellfunktion der CNRD(di)-Koeffizienten, derart, daß:

$$CNRD(d) = A'_0 + A'_1 \log\frac{1}{d} + A'_2 \left[\log\frac{1}{d}\right]^2 \quad (20)$$

wobei $A'_0$, $A'_1$ und $A'_2$ die Parameter sind, die anhand der Meßpunkte geschätzt werden, wodurch die Bestimmung des einer gegebenen Filmrate entsprechenden Koeffizienten möglich ist.

8. Verfahren gemäß Anspruch 7, dadurch gekennzeichnet, daß die Filmrate d durch die Zelle (4, 12) gemessen wird.

9. Verfahren gemäß Anspruch 8, dadurch gekennzeichnet, daß die Filmrate $d_i$ durch die folgende Formel gegeben ist:

$$d_i = \frac{L_{ref} \times CNRT(t_i)}{t_i}. \quad (28)$$

10. Verfahren gemäß Anspruch 9, dadurch gekennzeichnet, daß die von der Aufnahmezeit $t_i$ abhängigen Koeffizienten $CNRT(t_i)$ dadurch erhalten werden, daß die folgenden Operationen ausgeführt werden:
   $(a_1)$ Modifizieren des Heizstroms der Röhre (11) in der Weise, daß verschiedene Werte des Stroms erhalten werden,
   $(a_2)$ Ablesen der Werte $M(t_i)$, die von der Integratorschaltung (16) für die verschiedenen Aufnahmezeiten $(t_i)$ ausgegeben werden, so daß eine optische Dichte $DO_1$ des Films erhalten wird,
   $(a_3)$ Berechnen des Verhältnisses

$$\frac{M(t_i)}{M(t_{ref})}, \quad (29)$$

das den Koeffizienten $CNRT(t_i)$ ergibt, wobei $M(t_{ref})$ der Wert $M(t_i)$ für $t_i = t_{ref}$ ist.

11. Verfahren gemäß Anspruch 9, dadurch gekennzeichnet, daß die Koeffizienten $CNRT(t_i)$ dadurch erhalten werden, daß die folgenden Operationen ausgeführt werden:
   (g1) Verwirklichen eines ersten Sensitogramms $S_{refo}$ mit Hilfe eines Sensitometers mit veränderlicher Zeit, wenn die Aufnahmezeit für eine Referenzzeit $t_{refo}$ gesteuert wird;
   (g2) Verwirklichen von q Sensitogrammen $S_1$ bis $S_q$ für q verschiedene Aufnahmezeiten $t_i$ mit Hilfe desselben Sensitometers mit veränderlicher Zeit;
   (g3) Wählen einer optischen Referenzdichte $DO_{refo}$,
   (g4) Messen der Beleuchtungsstufe $Ech_{refo}$, $Ech_1$,..., $Ech_i$, $Ech_q$, die der optischen Dichte $DO_{refo}$ entspricht, für jedes Sensitogramm,
   (g5) Berechnen des Koeffizienten $CNRT(t_i)$ durch die Gleichung

$$CNRT\ (ti)\ =\ \exp\left[\log_{10}\left[\frac{Ech_{refo}\ -\ Ech_i}{K}\right]\right] \qquad (28)$$

mit $K = 2/\log_{10}(2)$.

**12.** Verfahren gemäß Anspruch 10 oder 11, dadurch gekennzeichnet, daß es außerdem eine Operation der Modellbildung der Koeffizienten $CNRT(t_i)$ in Form eines analytischen Modells enthält:

$$CNR\ T(t)\ =\ A_0\ +\ A_1\ \log t\ +\ A_2[\log t]^2 \qquad (18)$$

wobei $A_0$, $A_1$ und $A_2$ Parameter sind, die anhand der Meßpunkte geschätzt werden.

**13.** Verfahren gemäß einem der Ansprüche 1 bis 12, dadurch gekennzeichnet, daß die Operation (a") der Eichung der Referenzbelichtung die folgenden Operationen enthält:
- Verwirklichen einer Aufnahme unter bestimmten radiologischen Bedingungen für eine optische Referenzdichte, beispielsweise $DO_{refo} = 1$, ein Normal der Dicke $E_o$, eine Versorgungsspannung $V_o$ und einen Wert des Produkts eines Anodenstroms $I_o$ der Röhre mit einem Wert der Aufnahmezeit $t_o$;
- Berechnen des Wirkungsgrades $D_{fo}$ durch die Einrichtung (18);
- Berechnen der Belichtung $L_{Film}$ für den Film durch die Formel
$$L_{film}\ =\ D_{fo}\ \times\ I_o\ \times\ t_o, \qquad (23)$$
- Berechnen der Beleuchtungsstufe $Ech_{ref}$, die der optischen Referenzdichte $DO_{refo}$ entspricht, mit Hilfe der sensitometrischen Kurve;
- Messen der optischen Dichte $DO_m$ der erhaltenen Aufnahme und Berechnen der Beleuchtungsstufe $Ech_m$ mit Hilfe der sensitometrischen Kurve und
- Berechnen der Referenzbelichtung $L_{ref}$ durch die Formel:

$$L_{ref} = L_{film}\ \times \exp\left[\log_{10}\left[\frac{Ech_{ref} - Ech_m}{K}\right]\right] \qquad (25)$$

mit

$$K\ =\ 2/\log_{10}(2). \qquad (26)$$

**14.** Verfahren gemäß den Ansprüchen 12 und 13, dadurch gekennzeichnet, daß die korrigierte Belichtung für den Film $L'_{Film}$ durch die Formel berechnet wird:

$$L'_{film}\ =\ \frac{D_{fo}\ \times\ I_o\ \times\ t_o}{CNRT(t_o)}. \qquad (23')$$

**15.** Verfahren gemäß Anspruch 13 oder 14, dadurch gekennzeichnet, daß eine korrigierte Belichtung $L_{cvn}$ gemäß der Formel berechnet wird:

$$L_{cvn} = L_{ref}\ \times \exp\left[\frac{CVN}{\Gamma}\times P \times \log(10)\right], \qquad (27)$$

so daß eine unterschiedliche Schwärzung (oder optische Dichte) erhalten wird, wobei in der Formel:
- CVN die gewünschte Korrektur der Schwärzung ist, die beispielsweise durch eine ganze Zahl ausgedrückt wird,
- P die Schrittweite der optischen Dichte ist,
- $\Gamma$ die Steigung des linearen Teils der sensitometrischen Kurve ist.

## Claims

**1.** A method for the automatic determination of the duration of exposure of a radiographic film in a radiology system provided to examine an object (13), which comprises an x-ray tube (11) whose supply voltage may assume various different values $v_m$, with continuous or discrete variation, and which emits a beam (14) of x-rays in the form of pulses with a variable duration S toward the object (13) to be examined, a receiver (17) for x-rays which have passed through the object (13) in order to produce an image of the said object, the said receiver being made up of at least one intensifier screen (2) and of a film (3) sensitive to the light emitted by the said screen, a detection cell (4), which is provided in order to detect the light rays emitted by the said screen (2), arranged in the said receiver downstream from the said screen, and which permits the conversion of a physical quantity characteristic of the x-ray beam into a measurement signal L, an integrating circuit (16) which integrates the measurement signal L during the duration S and supplies a signal M, and a calculation device (18) for a yield D equal to the ratio of M to the product of the anode current I of the tube times the duration S of exposure I $\times$ S (or mA.s), such method comprising the following operations:

(a") calibration in order to determine the reference exposure radiation $L_{ref}$ which is to be received by the film, under predetermined reference conditions in order to arrive at the blackening (or optical density) selected as a reference value by the user;

(b1) putting the object (13) to be radiographed in position;

(b2) causing the start of exposure by the user;

(b3) measurement of the yield $D_{f1}$ a certain t' after the start of exposure or, if appropriate, the last measurement;

(b4) calculation of the lumination $I_f$ received by the film in accordance with the equation

$$L_f = L_{am} + D_{f1} \times \delta mA.s \quad (9)$$

wherein $L_{am}$ is the lumination received by the film prior to the operation (b3) and $\delta mA.s$ is the number of mA.s supplied during the time in the time t' and is defined by the product of the current I of the tube times the integration time f';

(b5) calculation of the lumination $L_{ra}$ remaining to be acquired in order to obtain a selected blackening (or optical density) by the equation:

$$L_{ra} = L_{ref} - L_f \quad (10)$$

(b6) a forecasting calculation of mM.s remaining to be supplied ($mAs_r$) in order to obtain the selected blackening (or optical density) by the equation:

$$mAs_r = \frac{L_{ra}}{D_{f1}} \quad (11)$$

(b7) measurement of mA.s ($mAs_{mes}$) as from the start of exposure or, if appropriate, of the preceding measurement;

(b8) stopping the exposure when the measurement $mAs_{mes}$ becomes equal to or greater than

$$mAs_r = \frac{L_{ra}}{D_{f1}} \quad (11)$$

- or a return to the operation (b3) when the measurement $mAs_{mes}$ in (b7) is less than $mAs_r$.

2. A method for the automatic determination of the duration of exposure of a radiographic film in a radiology system provided to examine an object (13), which comprises an x-ray tube (11) whose supply voltage V may assume various different values $v_m$, with continuous or discrete variation, and which emits a beam (14) of x-rays in the form of pulses with a variable duration S toward the object (13) to be examined, a receiver (17) for x-rays which have passed through the object (13) in order to produce an image of the said object, the said receiver being made up of at least one intensifier screen and of a film sensitive to the light emitted by the said screen, a detection cell (12) for x-rays which has passed through the object to be examined, arranged behind the said image receiver (17) to permit the conversion of a physical quantity characteristic of the x-ray beam (14) into a measurement signal L, an integrating circuit (16) which integrates the measurement signal L during the duration S and supplies a signal M, and a calculation device (18) for the yield D equal to the ratio of M to the product I $\times$ S (or mA.s), of the anode current I of the tube times the duration S of exposure S, such method comprising the following calibration operations:

(a) a first calibration of the radiology system with the aid of objects with a thickness $E_p$ utilizing a receiver without the intensifier screen or screens in such a manner as to determine the yield function $D_{se}$ such that:

$$D_{se} = f' (V_m, E_p) \quad (4)$$

and the inverse function

$$E_p = g' (V_m, D_{se}) \quad (5)$$

(b) a second calibration of the radiology system with the aid of objects with a thickness $E_p$ utilizing the said receiver (17) with the intensifier screen or screens in such a manner as to determine the yield function $D_c$ such that:

$$D_c = f'' (V_m, E_p) \quad (6)$$

and the inverse function

$$E_p = g'' (V_m, D_c) \quad (7)$$

and the calculation of the yield function $D_f$ such that :

$$D_f = f' (V_m, E_p) - f'' (V_m, E_p) \quad (8)$$

wherein $f'$ and $f''$ are the functions which describe the variation of the yield D as a function of the parameters $V_m$ and $E_p$ for the given configurations of the system,

(c) a third calibration in order to determine the reference lumination $L_{ref}$ to be received by the film under predetermined reference conditions, in order to arrive at the blackening (or optical density) selected as a reference value by the user; and then the following operations:

(e1) the putting into position of the object (13) to be radiographed;

(e2) causing the start of exposure by the user;

(e3) measurement of the yield $D_{c1}$ a certain $t'$ after the start of exposure or, if appropriate, the last measurement;

(e4) measurement of the equivalent thickness $E_1$ in accordance with the equation (7);

(e5) calculation of the yield $D_{f1}$ at the level of the film for the thickness $E_1$ by the equation (8);

(e6) calculation of the lumination $L_f$ received by the film in accordance with equation

$$L_f = L_{am} + D_{f1} \times \delta mA.s \quad (9)$$

wherein $L_{am}$ is the lumination received by the film prior to the operation (e3) and $\delta mA.s$ is the number of mA.s supplied in the time $t'$ and is defined by the product of the current I of the tube times the integration time $f'$;

(e7) calculation of the lumination $L_{ra}$ remaining to be acquired in order to obtain a selected blackening (or optical density) by the equation:

$$L_{ra} = L_{ref} - L_f \quad (10)$$

(e8) a forecasting calculation of mA.s remaining to be supplied $mAs_{ra}$ in order to obtain the selected blackening (or optical density) by the equation:

$$mAs_r = \frac{L_{ra}}{D_{f1}} \quad (11)$$

(e9) measurement of mA.s supplied as $mAs_{mes}$ as from the start of the operation (e3) or, if appropriate, of the preceding exposure;

(e10) stopping the exposure when the mA.s measured as $mAs_{mes}$ in step (e9) is equal to or greater than $mAs_r$.

- or a return to the operation (e3) when mA.s measured in (e9) are less than $mAs_r$.

3. The method as claimed in claim 1 or claim 2, characterized in that it furthermore comprises,

- an operation for the conversion of $mAs_{ra}$ into a target value $CE_{target}$ in the units of the cell (4) such that :

$$CE_{target} = mAs_r \times D_{f1}$$

- an operation of decrementing the target value $CE_{target}$ by signals received from the detection cell (12; 4) in such a manner as to stop the exposure when the decremented value becomes less than or equal to a predetermined value $Val_0$.

4. The method as claimed in claim 1, characterized in that the operation (b6) furthermore comprises a calculation operation for the mA.s ($mAs_c$) supplied during the calculation operations (b4) through (b6) of a duration $t_c$ using the equation:

$$mAs_c = I \times t_c \quad (13)$$

in such a manner as to determine the real value of mA.s remaining to be acquired ($mAs_{r0}$) such that:

$$mAs_{ra} = mAs_r - mAs_c \quad (12).$$

5. The method as claimed in claim 4, characterized in that the operation for the calculation of $mAs_r$ is periodically repeated during the exposure at instants $t_1$, $t_2$...$t_n$ separated by a duration which is at least equal to the calculation time $t_c$ of the operations (b4) through (b6).

6. The method as claimed in claim 1, characterized in that corrected calculations for $L_f$ and for $L_{ra}$ are performed respectively in accordance with the following equations:

$$L''_f = L_{am} + D_{f1} \times \frac{\delta mA \cdot s}{CNRD(film\,rate)} \quad (9')$$

and

$$mAs''_{ra} = \frac{L_{ra}}{D_{f1}} \times CNRD(\text{film rate}) \quad (11')$$

in which equations CNRD (film rate) represents the coefficient of non-reciprocity indexed as the film rate of the receiver (17) such that

$$\text{film rate} = D_{f1} \times I \quad (17).$$

7. The method as claimed in claim 6, characterized in that the CNRD (film rate) coefficient is obtained by performing the following operations:
   - measurement of the non-reciprocity coefficients CNRT $(t_i)$ of the film-screen system as a function of the exposure time $t_i$,
   - measurement for each exposure time $(t_i)$ of the film rate $t_i$,
   - determination of the modeling function of the coefficients CNRD (di) such that:

$$CNRD(d) = A'_0 + A'_1 \log\frac{1}{d} + A'_2\left[\log\frac{1}{d}\right]^2 \qquad (20)$$

   wherein $A'_o$, $A'_1$ and $A''_2$ are parameters which are estimated on the basis of the measurement points, this permitting the determination of the coefficient corresponding to a given film rate.

8. The method as claimed in claim 7, characterized in that the film rate is measured by the cell (4, 12).

9. The method as claimed in claim 8, characterized in that the film rate $d_i$ is given by the equation

$$d_i = \frac{L_{ref} \times CNRT(t_i)}{t_i}. \quad (28)$$

10. The method as claimed in claim 9, characterized in that the coefficients CNRT $(t_i)$ as a function of the exposure time $t_i$ are obtained by performing the following operations.
    $(a_1)$ the modification of the heating current of the tube (11) in such a manner that different values of the said current are obtained,
    $(a_2)$ taking the values M $(t_i)$ furnished by the integrating circuit (16) for different exposure times $(t_i)$ in such a manner as to obtain the optical density $DO_1$ of the film,
    $(a_3)$ calculation of the ratio

$$\frac{M(t_i)}{M(t_{ref})}, \quad (29)$$

    which gives the coefficient CNRT $(t_i)$ with M $(t_{ref})$ the value M $(t_i)$ being $t_i = t_{ref}$.

11. The method as claimed in claim 9, characterized in that the coefficients CNRT $(t_i)$ are obtained by performing the following operations:
    (g1) the production with the aid of a variable time sensitographic instrument of a first sensitogram $s_{refo}$ when the exposure time is regulated for a reference time $t_{refo}$;
    (g2) the production, with the aid of the same variable time sensitographic instrument, of q sensitograms $s_1$ to $s_q$ for q different exposure times $t_i$;
    (g3) the selection of a reference optical density $DO_{refo}$,
    (g4) the measurement using each sensitogram of the illumination increment $ECh_{refo}$, $Ech_1$,...$ECh_i$...$Ech_q$ corresponding to the optical density $DO_{refo}$,
    (g5) the calculation of the coefficient CNRT $(t_i)$ by the equation:

$$CNRT\ (ti) = \exp\left[\log_{10}\left[\frac{Ech_{refo} - Ech_i}{K}\right]\right] \qquad (28)$$

    wherein $K = 2/\log_{10}(2)$.

12. The method as claimed in claim 10 or claim 11, characterized in that it furthermore comprises a modeling operation for the coefficients CNRT $(t_i)$ in the form of the analytical model:

$$CNRT(t) = A_0 + A_1 \log t + A_2 [\log t]^2 \quad (18)$$

$A_0$, $A_1$ and $A_2$ being parameters which are estimated on the basis of measurement points.

13. The method as claimed in any one of the claims 1 through 12, characterized in that the calibration operation (a") for the reference lumination comprises the following operations:
- the production of an exposure under radiological conditions predetermined for a reference optical density, for example $DO_{refo} = 1$, of a standard of a thickness $E_o$, a supply voltage $V_o$ and a value of the product of an anode current $I_o$ of the tube times a value $t_o$ of the exposure time;
- calculation of the yield $D_{fo}$ by the device (18),
- calculation of the luminance Lfilm on the film in accordance with the equation:
$$L_{film} = D_{fo} \times I_o \times t_o \quad (23)$$
- calculation of the illumination increment $Ech_{ref}$ corresponding to the reference optical density $DO_{refo}$ with the aid of the sensitometric curve;
- measurement of the optical density $DO_m$ of the exposure obtained and calculation of the illumination increment $Ech_m$ with the aid of the sensitometric curve, and
- calculation of the reference illumination $L_{ref}$ by the equation:

$$L_{ref} = L_{film} \times \exp\left[\log_{10}\left[\frac{Ech_{ref} - Ech_m}{K}\right]\right] \qquad (25)$$

wherein
$$K = 2/\log_{10}(2) \quad (26).$$

14. The method as claimed in claims 12 and 13, characterized in that the corrected brightness $L'_{film}$ is calculated by the equation;

$$L'_{film} = \frac{D_{fo} \times I_o \times t_o}{CNRT(t_o)} . \quad (23')$$

15. The method as claimed in claim 13 or claim 14, characterized in that a corrected lumination $L_{cvn}$ is calculated using the equation:

$$L_{cvn} = L_{ref} \times \exp\left[\frac{CVN}{\Gamma} \times P \times \log(10)\right], \qquad (27)$$

in such a manner as to obtain a different blackening (or optical density) in which equation:
- CVN is the arbitary correction of blackening expressed for example by a whole number,
- P is the step of the optical density,
- $\Gamma$ is the slope of the linear part of the sensitometric curve.

**FIG. 1**

**FIG. 2**

FIG. 3

FIG. 4

# FIG. 5

FIG. 6